# EUROPEAN PATENT SPECIFICATION

(11) **EP 0 424 473 B1**
(45) Date of publication and mention of the grant of the patent: **08.05.1996**
(21) Application number: 89909671.3
(22) Date of filing: 30.06.1989
(51) Int. Cl.: C12Q 1/68, C12N 15/00, C07H 15/12

(54) **METHODS FOR IDENTIFICATION OF BACTERIA**
VERFAHREN ZUR IDENTIFIZIERUNG VON BAKTERIEN
PROCEDES D'IDENTIFICATION DE BACTERIES

(30) Priority: 05.07.1988 US 215135
(43) Date of publication of application: 02.05.1991
(73) Proprietor: BAYLOR COLLEGE OF MEDICINE, Houston, TX 77030 (US)
(72) Inventor: LUPSKI, James, R., Houston, TX 77030 (US)
(74) Representative: Adams, William Gordon
(86) International application number: US8902884
(87) International publication number: WO9000624

(56) References cited:
- EP-A- 0 224 294
- WO-A-83/01451
- WO-A-87/05907
- WO-A-87/07300
- US-A- 4 358 535
- US-A- 4 740 463
- US-A- 4 801 540
- SCIENCE, vol. 242, 18 November 1988; K. TANAKA et al., pp. 1040-1042
- JOURNAL OF BACTERIOLOGY, vol. 169, no. 7, 30 June 1987; AIBA et al., pp. 3007-3008
- NUCLEIC ACIDS RESEARCH, vol. 14, no. 10, 1986; WANG et al., pp. 4293-4295
- PROCEEDINGS OF THE NATL. ACADEMY OF SCIENCES USA, vol. 80, February 1983, Washington, DC (US); KONIGSBERG et al., pp. 687-689
- GENE, vol. 40, 1985; ERICKSON et al., pp. 67-69
- CELL, vol. 42, August 1985; KIM et al., pp. 129-131
- CELL, vol. 39, December 1984; LUPSKI et al., pp. 251-252
- TRENDS IN BIOCHEMICAL SCIENCE, vol. 9, no. 11, November 1984; LAPORTE, p. 463
- MOLECULAR & GERERAL GENETICS (MGG), vol. 195, 1984; LUPSKI et al., pp. 391-393
- PROCEEDINGS OF THE NATL. ACADEMY OF SCIENCES USA; vol. 80, July 1983, Washington, DC (US); LEARY et al., pp. 4045-4049

## Description

The present invention relates generally to antisense oligonucleotides which bind to a messenger RNA and single strand DNA. More particularly it relates to antisense oligonucleotides which bind to messenger RNA transcribed from the macromolecular synthesis operon of bacteria.

It further relates to the method of identification of bacteria by the binding of an antisense oligonucleotide specifically to a unique sequence in the intergenic regions of the macromolecular synthesis operon of bacteria.

It has been demonstrated that the genes involved in initiating the synthesis of DNA, RNA and protein in bacteria are contained in one single structural unit name the macromolecular synthesis operon (MMS). The genes are part of a single transcription unit and have been identified as rpsU encoding ribosomal protein S21 involved in initiating translation, dnaG encoding the protein primase which initiates DNA replication and rpoD which encodes sigma-70 involved in initiating transcription. The operon structure is found in both gram negative bacteria, such as Escherichia coli and Salmonella typhimurium, and in gram positive bacteria such as Bacillus subtilis. The individual structural genes are conserved and have large areas of homology. On the other hand, the intergenic sequences between the structural gene within the operon are unique to each bacterial species. The MMS operon appears to be a central information processing unit for directing the flow of genetic information. The organization of the operon suggests that under certain physiological conditions there is a need for coordination of synthesis of the information macromoleculas (DNA, RNA and protein) in the cell and hence a coregulation of the initiator genes. Since the synthesis of each class of macromolecule appears to be regulated at its initiation step, regulation of the MMS operon most likely plays a role in regulating cell growth.

The MMS operon contains three structural genes. The rpsU gene encodes the ribosomal protein S21 which is required for specific initiation of messenger RNA (mRNA) translation. The protein S21 interacts with a stretch of ribosomal RNA (rRNA) complementary to the mRNA ribosomal binding site called the Shine-Dalgarno sequence located at the 3' end of the 16S rRNA. Colicin E3 removes 50 nucleotides from the 3' terminus of 16S rRNA. E3 treated ribosomes cannot carry out polypeptide chain initiation nor chain elongation. In reconstitution experiments, E3 treated ribosomes bind all 30S proteins except S21. RNA protein cross-linking experiments demonstrate that protein S21 is cross-linked to the 3' dodecanucleotide of the 16S rRNA. The base-pairing potential of the 3' terminus of 16S rRNA depends on the functional state of the 30S subunit and the presence of S21, which is required for specific initiation of E. coli and phage MS2 mRNA translation.

Initiation of DNA replication requires a priming RNA which is synthesized by the dnaG gene product, primase. This protein binds to the phage G4 origin of replication. Primase also is known to interact with the multienzyme complex primosome to initiate synthesis of Okazaki fragments on the chromosomal replication fork-lagging strand of E. coli. Primase is the sole priming enzyme required for initiation of DNA replication at the origin of the E. coli chromosome. A parB mutation in the dnaG gene results in abnormal partition of chromosomes and was originally isolated as a thermosensitive mutant affecting DNA synthesis and cellular division. Thus, in addition to initiation of DNA replication, the dnaG gene appears to play some role in regulating cell division.

The rpoD gene product sigma-70 is involved in the recognition of promoter sequences for the specific initiation of RNA transcription. Sigma-70 interacts with the core polymerase α₂ββ' conferring specificity for promoter sequences. Sigma-70 is a member of a large family of RNA polymerase sigma factors. Thus, the macromolecular synthesis operon gene products share a common mechanism. Through protein-nucleic acid interactions the gene products of the MMS operon bind specific nucleotide sequences. For example S21 binds the Shine-Dalgarno sequence/ribosome binding site, primase binds the origin of replication, and sigma-70 binds a promoter sequence. These interactions result in initiation of synthesis of protein, DNA or RNA respectively.

Antisense RNAs have been utilized both in nature and experimentally to regulate gene expression. For example antisense RNA is important in plasmid DNA copy number control, in development of bacteriophage P22. Antisense RNAs have been used experimentally to specifically inhibit in vitro translation of mRNA coding from Drosophila hsp23, to inhibit Rous sarcoma virus replication and to inhibit 3T3 cell proliferation when directed toward the oncogene c-fos. Furthermore, it is not necessary to use the entire antisense mRNA since a short antisense oligonucleotide can inhibit gene expression. This is seen in the inhibition of chloramphenicol acetyltransferase gene expression and in the inhibition of specific antiviral activity to vesicular stomatitus virus by inhibiting the N protein initiation site. Antisense oligonucleotides to the c-myc onocogene have been demonstrated to inhibit entry into the S phase but not the progress from G_{O} to G₁. Finally, inhibition of cellular proliferation has been demonstrated by the use of antisense oligodeoxynucleotides to PCNA cyclin.

CELL, Vol. 42, Aug. 1985 teaches the use of cloned and expressed anti-sense RNA (asRNA) to reduce the level of thymidine kinase activity in L cells. TRENDS IN BIOCHEMICAL SCIENCE, Vol. 9, No. 11, Nov. 1984 similarly teaches the use of asRNA as a means to regulate or interrupt gene expression at the level of translation by bind to complementary mRNA. Each one of CELL, Vol. 39, Dec. 1984 and MOLECULAR AND GENERAL GENETICS (MGG), Vol. 195, 1984 (LUPSKI ET AL) teach the macromolecular (MMS) operon of E. coli and the regulation of expression thereof. Additionally, LUPSKI ET AL (MGG 1984) teaches the use of asRNA as a means of regulating the expression of the E. coli MMS operon. Each one of GENE, Vol. 40, 1985 and NUCLEIC ACIDS RESEARCH, Vol. 14, No. 10, 1986 teach the MMS operons of Salmonella typhimurium and B. subtilis, respectively, and the comparison thereof with the MMS operon of E. coli.

However the method of employing intergenic oligonucleotides to identify bacteria is not taught or fairly suggested by the prior art or any combination thereof.

The present invention provides antisense oligonucleotide sequences which bind mRNA transcribed from the MMS operon or a unique intergenic sequence of the MMS operon.

An object of the present invention is a method for identifying bacteria.

Another object of the present invention is the provision of a sequence which detects the presence or absence of bacteria.

Thus, in accomplishing the foregoing objects there is provided in accordance with one aspect of the present invention a method of identifying bacteria comprising the steps of hybridizing an antisense oligonucleotide to a mRNA transcribed from said macromolecular synthesis operon and measuring the amount of said hybridization. The antisense oligonucleotide sequence can be specific to a unique intergenic sequence in the mRNA or it can be a sequence which is specific to a region of the mRNA containing a sequence which is homologous between bacterial strains or any combination of these.

In preferred embodiments, the antisense oligonucleotide antibiotic can be selected from the following sequences:

5' CATCCAAAGCAGTGGTAAAACTGTTT 3',
5' TCACCGATCGGCGTTTCCA 3', 5' GGCCCCGATTTTTAGCAA 3',
5' CTTGCGTAAGCGCCGGGG 3' and 5' TATTCGATGCTTTAGTGC 3'.

Another aspect of the present invention is a method for typing or identifying bacteria comprising the steps of binding a unique intergenic antisense oligonucleotide to a mRNA transcribed from the macromolecular synthesis operon and then determining the amount of binding between the species specific macromolecular synthesis oligonucleotide and the mRNA transcribed from the macromolecular synthesis operon of a given bacterial species.

A further aspect of the present invention is the use of a homologous sequence to detect the presence or absence of bacteria.

In the identification of bacteria the antisense oligonucleotide is at least 10 nucleotides (10 mer). In a preferred embodiment, an oligonucleotide of 16 to 26 mer is used.

In another embodiment the 3' and/or 5' termini of the oligonucleotide is derivatized to prevent the degradation, e.g. by exonucleases, of the oligonucleotide after bacteria uptake.

Other and further objects, features and advantages will be apparent from the following description of the presently preferred embodiments of the invention given for the purpose of disclosure when taken in conjunction with the accompanying drawings, in which:-

Figure 1 is the macromolecular synthesis operon shown in schematic form. It contains three genes, one each, involved in the initiation of translation *(rpsU)*, replication *(dnaG)* and transcription *(rpoD).*

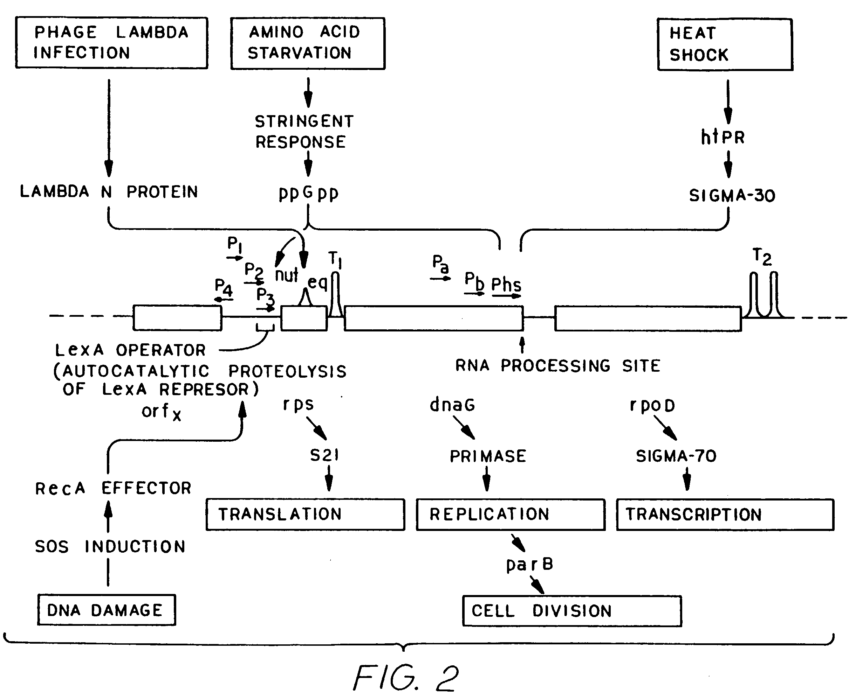

Figure 2 depicts the regulation of the *E. coli* macromolecular synthesis operon. The three genes in the macromolecular synthesis operon are depicted as closed boxes. The *cis*-acting regulatory sequences include promoters (Pₓ, P₁, P₂, P₃, Pₐ, P_{b}, Pₕₛ), terminators (T₁ and T₂), a LexA binding site, *nut*_{eg} and an RNA processing site. The *trans* acting factors are shown with arrows drawn to where they are believed to act. The NusA protein increases rpoD gene expression, but its site of action is unknown. Global regulatory networks that interact with the macromolecular synthesis operon include the SOS, heat shock and stringent response. A functional role for *orf*ₓ has not been assigned, but the proximity of Pₓ and the conservation of the *orf*ₓ sequences in *E. coli* and *S*. *typhimurium* suggests a possible macromolecular synthesis operon regulatory role. There are several other open reading frames further upstream with no assigned function and the nearest gene mapped on the E. coli chromosome is the cca gene which is 14 kb away.

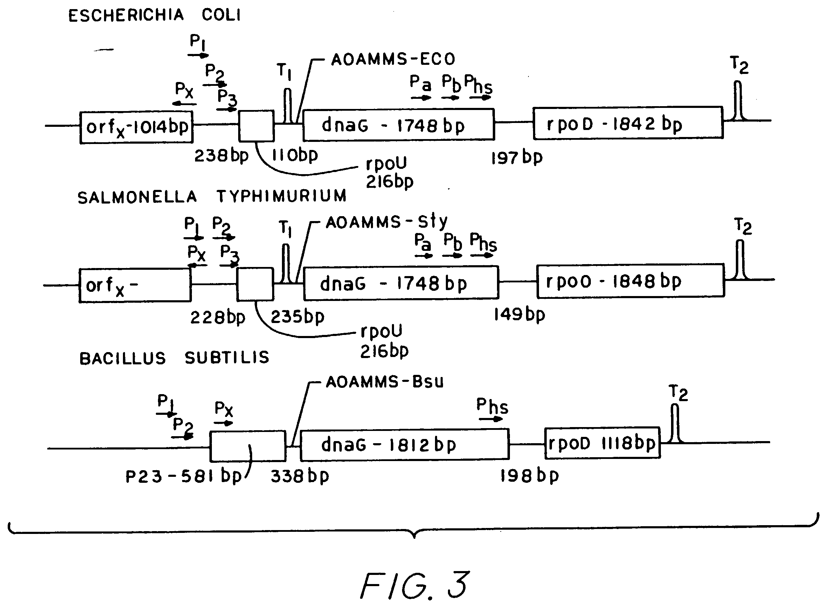

Figure 3 is a comparison of the MMS operon in different species. The structure of the MMS operon has been determined for E. coli, S. typhimurium and B. subtilis. The genes are depicted by open boxes with the size given in base pairs (bp) including termination codon. The size of the intergenic sequences is given below. Position of promoters (P) are denoted. AOAMMS - Eco is complementary to the E. coli MMS operon rpsU-dnaG intergenic sequences. AOAMMS - Sty is complementary to the S. Typhimurium MMS operon rpsU-dnaG intergenic sequences. AOAMMS - Bsu is complementary to the B. subtilis MMS operon rpsU-dnaG intergenic sequences.

Figure 4 shows a 5' modified antisense oligonucleotide antibiotic containing the addition of leucine.

Figure 5 shows a 3' modified antisense oligonucleotide antibiotic.

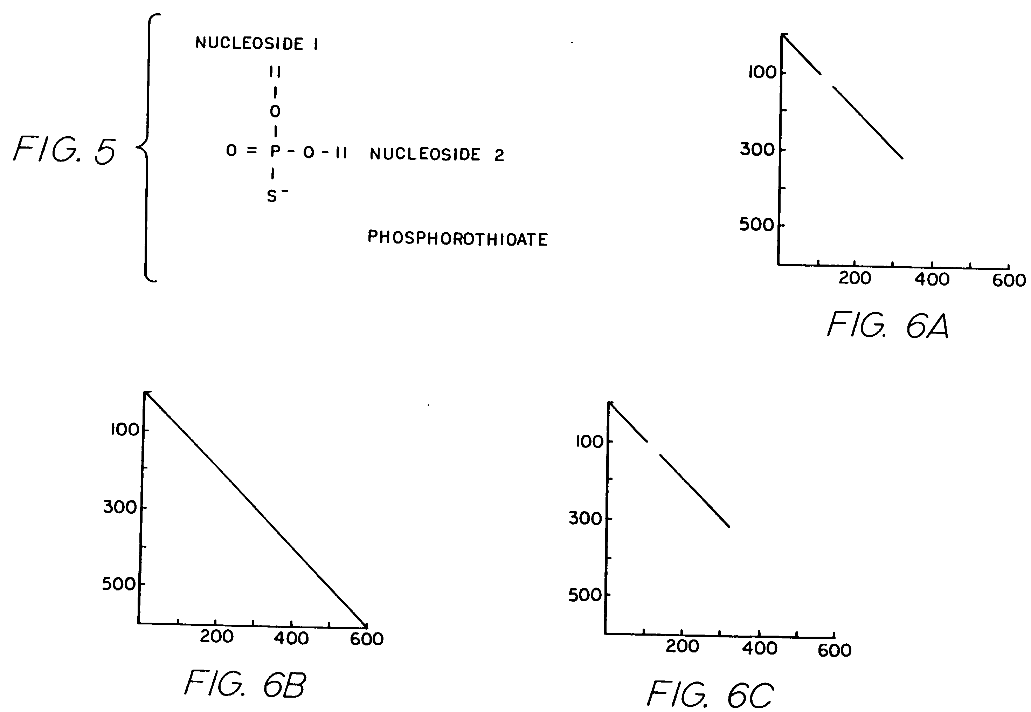

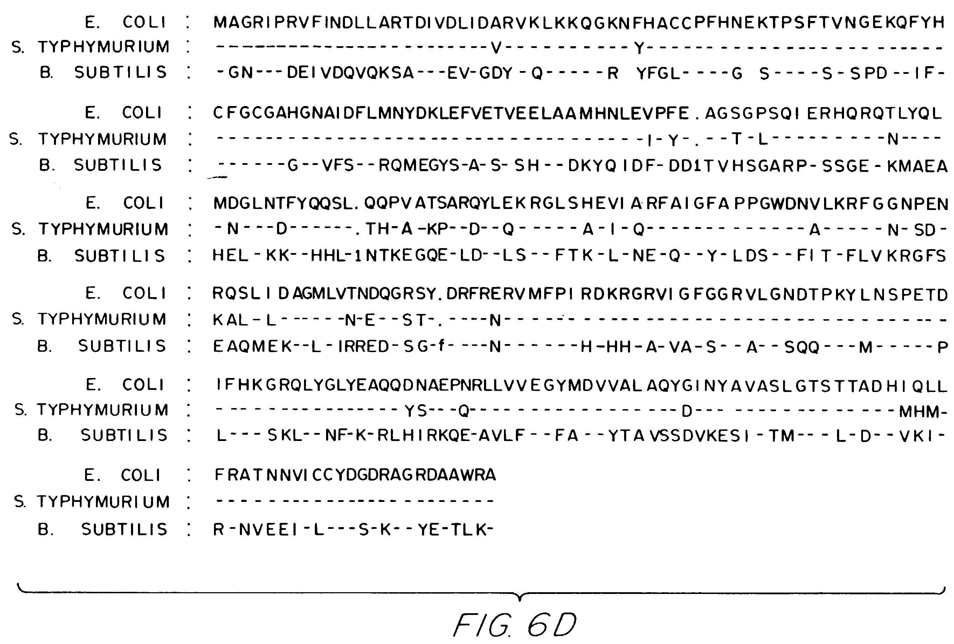

Figure 6 shows the homologies between bacterial strains for the primase gene. The information was generated from DNA sequences in GenBank utilizing the Molecular Biology Information Resources Multialign program to optimize homology searches of protein sequence data. The data is aligned from left to right on the abscissa, the amino terminal to the carboxy terminal portions of the protein. The numbers represent the amino acid positions in the protein primary sequence. In (a) B. subtilis was compared to E. coli, while in (b) S. typhimurium was compared to E. coli, and in (c) B. subtilis is compared to S. typhimurium. In (d), the S. typhimurium and B. subtilis primase protein sequences have been aligned to the E. coli dnaG primase in the amino terminal region. Upper case letters represent aligned non-identical amino acids while lower case letters signify non-aligned amino acids. The dashes represent aligned identical bases while the dots signify gaps. The data demonstrate that the primase proteins are related and share homology domains particularly in the amino terminal regions. The nucleotide sequence encoding these areas of amino acid homology are also very homologous.

Figure 7 is a picture of 1% agarose gel showing antisense binding of MMS operon probe sequences to restriction digested purified chromosomal DNA by Southern blotting.

The drawings are not necessarily to scale and certain features of the invention may be exaggerated in scale or shown in schematic form in the interest of clarity and conciseness.

The MMS operon includes genes involved in initiating: translation, *rpsU*; replication, *dnaG*; and transcription, *rpoD.* These genes are contained within a single transcriptional unit, Figures 1 and 2, and are involved in initiating synthesis of the major information macromolecules of the cell. The organization of the operon suggests that under certain physiological conditions there is a need for coordination of synthesis of DNA, RNA and protein in the cell and hence a coregulation of the initiator genes. Since the synthesis of each class of information macromolecule (DNA, RNA and protein) appears to be regulated at its initiation step, regulation of the MMS operon most likely plays a role in regulating cell growth.

In the MMS operon *cis*-acting regulatory sequences can occur within the coding regions. In gram-negative bacteria these include the *nut*_{eq} site within the *rpsU* structural gene and promoters Pₐ, P_{b}, and Pₕₛ in the dnaG structural gene. Promoter P₃ of the B. subtillis MMS operon is within this gene coding for P23. Other cis-acting regulatory sequences are located in the intergenic regions; terminator T₁ is located between rpsU and dnaG and an RNA processing site occurs in the dnaG-rpoD intergenic sequences. Thus, multiple cis-acting regulatory sequences allow discoordinate regulation as well as differential relative rates of individual gene expression within this operon structure.

Codon usage can affect relative amounts of individual gene expression. The presence of codon preference reflects the relative concentrations of isoaccepting tRNA species in the cell. The use of rare codons provides a means to ensure low level expression of regulatory genes. The dnaG gene contains greater than ten times the number of rare triplet codons as other E. coli genes and the absolute number of rare codons in the dnaG mRNA is similar to that of other control genes (e.g. lacI, trpR). Rare codons also occur in the S. typhimurium dnaG mRNA and the dnaE gene of B. subtilis. An additional translational regulatory mechanism operative in the MMS operon relies on the occurrence of ribosome binding sites with varying degrees of complementarity to the Shine-Dalgarno sequence. This can be seen in the E. coli dnaG gene, and is presumably due to the difference in free energy of binding leading to less efficient binding of the ribosome to the dnaG portion of the MMS mRNA. Both of these translational regulatory mechanisms, rare codon usage and altered ribosome binding affinity may partially explain the observed apparent discoordination of expression of the genes in this operon. The steady state relative abundances for the MMS operon protein products in the E. coli cell are 40,000 for S21, 50 for primase and approximately 3000 for sigma-70.

Comparative analysis of three sequenced MMS operons reveals several interesting features (Figure 3). All of the operons contain three open reading frames and transcription of the operons is initiated by several promoters at the 5' end. The major promoters have overlapping nucleotide sequences (-10 and -35 regions) and the cis-acting regulatory sequences appear to be clustered in small regions. Each operon contains a heat shock promoter (Pₕₛ) within the DNA replication initiation gene, dnaG or dnaE. The E. coli and S. typhimurium operons contain an open reading frame (orfₓ) upstream of the external promoters (P₁, P₂, P₃). Only 7 bp separate the -35 sequences of Pₓ and P₁ in E. coli while these sequences actually overlap in the S. typhimurium operon.

The central gene in the MMS operon is the one involved in initiating DNA replication. The dnaG gene product, primase has several activities which include (i) a protein-protein interaction with the primosome complex, (ii) a protein-nucleic acid interaction for recognition of the origin, (iii) an RNA polymerase activity to synthesize the primer RNA and (iv) a role in the partitioning of chromosomes as suggested by the parB mutation in the dnaG gene. There are no promoters which transcribe the dnaG gene directly. A 5' transcription terminator, poor ribosome binding site, occurrence of rare codons and clustering of rare codons are all mechanisms that maintain low level expression of this gene. Overexpression of the dnaG gene from a regulated promoter on an autonomously replicating plasmid kills the host cells. Evidence that regulation of dnaG expression directly affects cell growth comes from Tn5 mutagenesis data. A cloned dnaG gene with the MMS operon promoters intact, on a multicopy plasmid slows the growth rate of the host cell harboring it. After insertion of Tn5 into the dnaG promoter regions, presumably leading to decreased *dnaG* gene expression, growth rates return to control levels demonstrating that an increased *dnaG* expression can affect growth. Isolation of the *parB* mutation also suggests a direct role for *dnaG in* chromosome partitioning, cell division, and therefore, bacterial cell growth. The primase proteins encoded by the DNA replication initiation genes from the three sequenced MMS operons contain several regions of homology (Figure 6).

The MMS operon is under very complex regulatory control which, teleologically would be expected of a unit whose control is important to regulation of cell growth. In addition to the intrinsic complex regulation, the operon interacts with several global regulatory networks including heat shock, the stringent response, and SOS. This operon appears to have evolved ways to be regulated both as a single unit and as a group of independent units by strategic positioning of transcriptional and translational control signals. The fact that the operon is the same in *E. coli* and *S. typhimurium* and very similar in *B. subtilis* suggests there is a selective advantage to evolving such a structure.

The term "oligonucleotide" as used herein, defines a molecule comprised of more than three deoxyribonucleotides or ribonucleotides. Its exact length will depend on many factors relating to the ultimate function or use of the oligonucleotide. A fragment of a sequence is any molecule containing some smaller part of a larger molecule.

The term "homologous sequence" as used herein, defines a sequence within the MMS operon which has been conserved in bacterial species such that the sequence is nearly identical among a variety of species.

This sequence can be used to determine the presence or absence of bacteria.

The term "unique intergenic sequences" as used herein, defines a section of non-coding DNA which is positioned between the *rpsU-dnaG/E* and *dnaG/E-rpoD* genes within the MMS operon. In Figure 3 some examples of the location within the MMS operon of the unique intergenic sequences, AOAMMS-Sty, AOAMAS-Eco and AOAMMS-Bsu are shown. These MMS operon intergenic sequences are unique for each different species of bacteria. Thus, a specific sequence will be characteristic for a specific species of bacteria. Because of this uniqueness, the intergenic sequences can be used to identify the bacteria.

The term "antisense" is used herein, defines an oligonucleotide the sequence of which is complementary to the sense strand of the MMS operon. An antisense oligonucleotide will hybridize or bind (form a complex by Watson-Crick base pairing) in a complementary fashion to the messenger RNA molecule which has been transcribed from the MMS operon, as well as to a single stranded DNA of the MMS operon. The antisense oligonucleotide can be designed to bind to a unique intergenic sequence.

"Derivitizing" the oligonucleotide means altering the structure of the oligonucleotide to perform a specific function (e.g. (1) an addition to the 3' or 5' end to increase uptake into the cell; (2) blocking the 3' or 5' end to prevent exonucleolytic breakdown). This procedure may provide a more functional and stable oligonucleotide when it is in the bacteria. For example, the 3' end can be derivitized by adding a phosphorothioate linked nucleotide.

Some examples of sequences which are used to bind to the mRNA of the MMS operon are seen in Table 1.

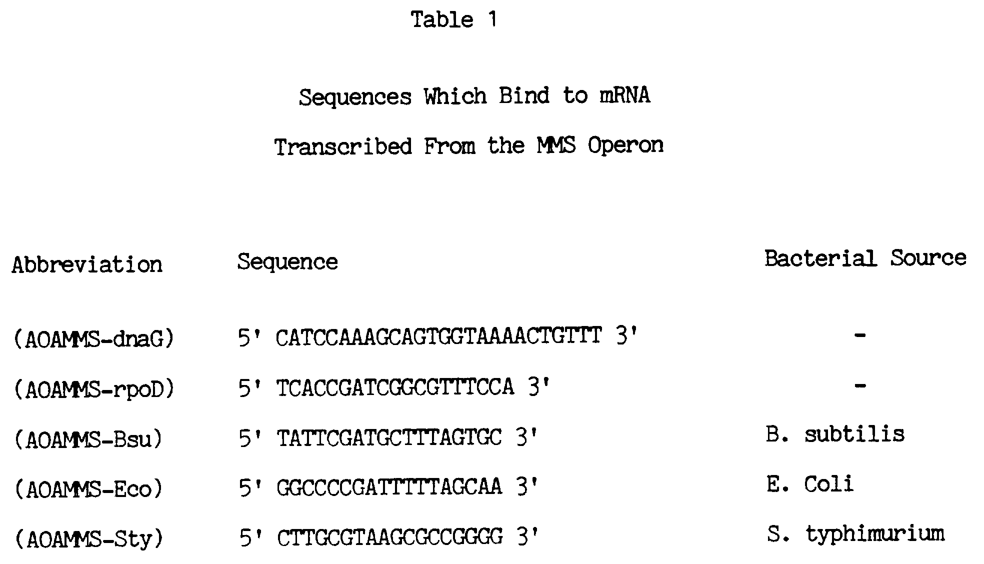

The sequences in Table 1 bind to bacterial homologous sequences are unique intergenic sequences which bind to specific sequences in specific bacteria. Each sequence is followed by the type of bacteria which is sensitive to the sequence.

In the preferred embodiment, using unique sequences, the nucleotide sequence of the proposed antisense oligonucloetide antibiotics is complementary to the intergenic region of the 5' side of the DNA replication initiation gene *(dnaG* or *dnaE)* (see Figure 3). This region of the MMS operon is chosen because the replication initiation gene has the lowest level of expression within the operon. Furthermore, in *E. coli* and *S. typhimurium,* this gene is located downstream from a terminator and is not directly transcribed by any promoter. In order to provide a more stable interaction with the mRNA, the primary sequences of the antisense oligonucleotide are chosen to maximize GC base pairing. However, one skilled in the art recognizes that there is a balance between maintaining the uniqueness of the sequence and maximizing the GC base pairing.

Another embodiment of the invention is a method of identifying bacteria comprising the steps of hybridizing a known species specific unique intergenic antisense oligonucleotide to a mRNA transcribed from a MMS operon or a single stranded DNA and measuring the amount of said hybridization to determine the type of bacteria. The unique sequence will only hybridize to a specific bacteria species, therefore no hybridization indicates a different species and hybridization indicates the species with the specific sequence. Each bacterial species contains a MMS operon with a unique intergenic sequence which can be used to uniquely identify each species. The mRNA which is transcribed from the MMS operon spans the whole operon and contains the homologous and unique intergenic sequences. By designing oligonucleotides which bind to the unique intergenic sequences, the diagnosis can be tailored to only identify a MMS operon in those bacterial species which have that unique sequence. Thus, by using a variety of antisense oligonucleotide probes, bacteria can be typed for each individual species. The amount of hybridization can be determined by a variety of methods known to those skilled in the art, including radioisotopes, enzymes, fluorescers, antibodies and chemiluminescers For example, the unique species specific intergenic antisense oligonucleotides can be labelled with biotin and then identified by a Strep avidin complex or a fluorescent tag.

The antisense oligonucleotides of Table 1 can be used to identify those bacteria which are listed after each antisense oligonucleotide sequence. One skilled in the art will readily recognize that as additional MMS operon intergenic sequences are sequenced, the present invention can be used to identify additional bacteria by antisense oligonucleotides synthesized to the unique intergenic sequences.

In bacteria typing, the length of the antisense oligonucleotide will be determined by the size necessary to bind specifically to the unique sequence. The oligonucleotide should be at least 10 nucleotides in length. In a preferred embodiment the sequences are between 16 and 26 mer.

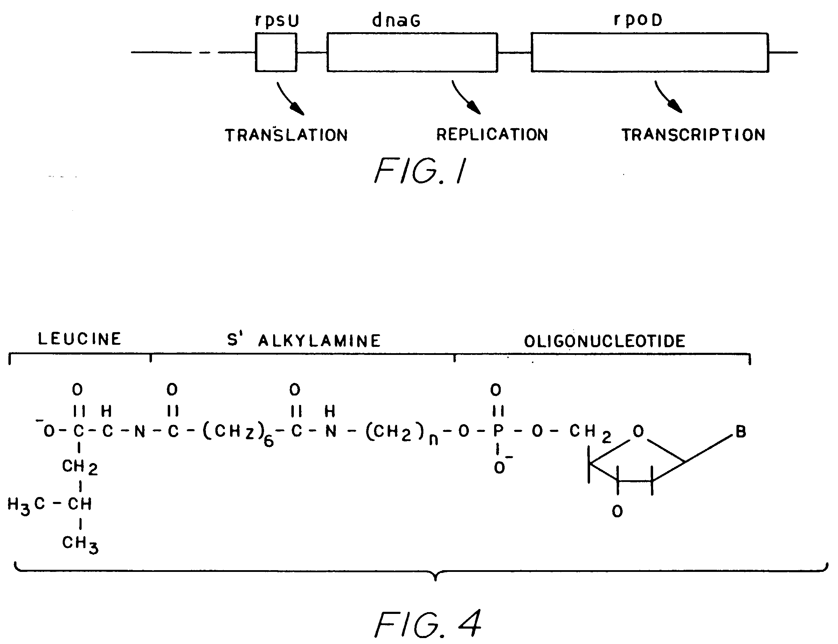

In Figure 4, the oligonucleotide is modified at the 5' end by adding a leucine molecule to the oligonucleotide. Bacteria have multiple transport systems for the recognition and uptake of molecules of leucine. The addition of this amino acid to the oligonucleotide facilitates the uptake of the oligonucleotide in the bacteria and does not interfere with the binding of the antisense oligonucleotide to the mRNA molecule.

One skilled in the art will readily recognize that other methods are available for facilitating the uptake of the antisense oligonucleotide antibiotic in the bacteria.

For example, addition of other amino acids enables utilization of specific transport systems.

Addition of lactose to the oligonucleotide by a covalent linkage can enable transport by lactose permease (product of the *lac* operon *Y* gene). Other sugar transport systems, known to be functional in bacteria, can be utilized to facilitate uptake into the bacterial cell.

Once an oligonucleotide with or without the carrier has entered the bacterial cell, it is important that it remain stable for the time period necessary to bind to the mRNA transcribed by the MMS operon. In one embodiment of the present invention, the oligonucleotide is derivatized at the 3' end to prevent degradation of the oligonucleotide (Figure 5). Other methods are known to alter the 3' and/or 5' ends of oligonucleotides to prolong the intracellular life and thus increase the availability for binding to the mRNA.

The expressed sequences or genes, *rpsU*, *dnaG, and rpoD,* within the MMS operon have regions that are conserved or homologous in all bacteria. These conserved homologous regions are utilized to identify the presence or absence of any bacteria by hybridizing an antisense ologonucleotide that identifies the conserved homologous sequences.

The intergenic regions are DNA sequences between the expressed sequences *rpsU-dnaG* and *dnaG-rpoD.* Intergenic sequences have not been conserved and thus are unique to a given bacterial species. Thus, these unique intergenic sequences are useful in identifying a particular species of bacteria.

To show that the expressed sequences within the MMS operon (rpsU, dnaG, rpoD) contain conserved homologous DNA sequences, the following oligonucleotide which recognized conserved DNA sequences within the dnaG gene.

AOAMMS - dnaG, 5'- CATCCAAAGCAGTGGTAAAACTGTTT-3' was synthesized: (sequence 1, Table 1)

This oligonucleotide was end labeled and used as a probe in Southern blotting. DNA was isolated from 12 different pathogenic strains of Salmonella obtained from the body fluids of infected patients, digested with HindIII and run on a 1% agarose gel. This digested chromosomal DNA was probed with the end-labeled dnaG oligonucleotide AOAMMS.

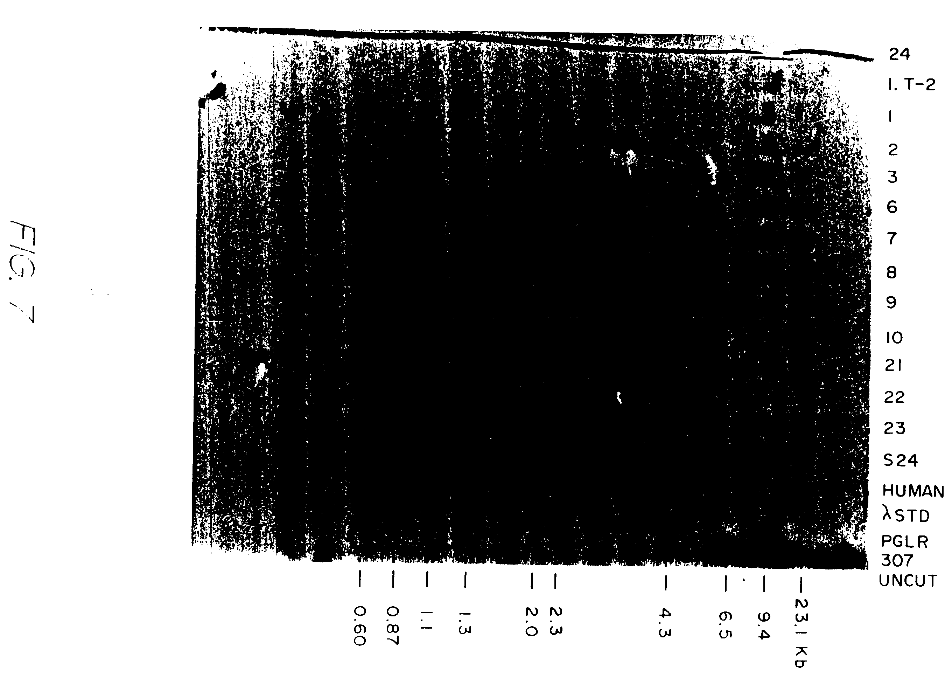

As seen in Figure 7, there is conservation of the oligonucleotide AOAMMS - dnaG in different pathogenic strains of Salmonella. The Southern blot shows homology of the oligonucleotide AOAMMS-dnaG to a laboratory control strain of Salmonella (LT-2) (lane 1) and twelve (12) different pathogenic strains isolated from body fluids of patients (lanes 2-13). There was no hybridization to human DNA (the negative control on lane 14), and as a positive control; a plasmid containing the DNA sequences in the probe showed a hybridization signal (lane 16). Lane 15 has lambda DNA cut with Hind III as a marker. On the far right are the sizes in kilobase pairs as determined on the agarose gel before Southern transfer.

One skilled in the art will readily appreciate that the present invention is well adapted to carry out the objects and obtain the ends and advantages mentioned, as well as, those inherent therein. The oligonucleotides, antibiotics, compounds, methods, procedures and techniques described herein are presently representative of preferred embodiments, are intended to be exemplary, and are not intended as limitations on the scope.

## Claims

1. A method of identifying bacteria, comprising the steps of:
hybridizing a unique intergenic antisense oligonucleotide of about 10 to 26 mer to a mRNA transcribed from a macromolecular synthesis operon; and
measuring the amount of said hybridization; wherein each unique intergenic antisense oligonucleotide only binds to a specific bacterium.

2. The method of claim 1, wherein the oligonucleotide is 5'GGCCCCGATTTTTAGCAA 3' and the bacteria is identified as E. coli.

3. The method of claim 1, wherein the oligonucleotide is 5'CTTGCGTAAGCGCCGGGG 3' and the bacteria is identified as S. typhimurium.

4. The method of claim 1, wherein the oligonucleotide is 5'TATTCGATGCTTTAGTGC 3' and the bacteria is identified as B. subtilis.

5. A method of identifying bacteria comprising the steps of:
treating a macromolecular synthesis operon to form single stranded DNA;
hybridizing an antisense oligonucleotide of about 10 to 26 mer to a unique intergenic sequence in the single stranded DNA of the macromolecular synthesis operon; and
measuring the amount of said hybridization.

6. The method of claim 5, wherein the oligonucleotide is 5'GGCCCCGATTTTTAGCAA 3' and the bacteria is identified as E. coli.

7. The method of claim 5, wherein the oligonucleotide is 5'CTTGCGTAAGCGCCGGGG 3' and the bacteria is identified as S. typhimurium.

8. The method of claim 5, wherein the oligonucleotide is 5'TATTCGATGCTTTAGTGC 3'; and the bacteria is identified as B. subtilis.

## Patentansprüche

1. Verfahren zur Identifizierung von Bakterien, welches die folgenden Schritte umfaßt:
Hybridisieren eines einzigartigen intergenischen Antisense-Oligonukleotides mit ca. 10 bis 26 Basen mit einer mRNA, die von einem makromolekularen Syntheseoperon transkribiert wurde; und
Messen des Ausmaßes der Hybridisierung; wobei jedes einzigartige intergenische Antisense-Oligonukleotid nur an ein bestimmtes Bakterium bindet.

2. Das Verfahren nach Anspruch 1, wobei das Oligonukleotid 5'GGCCCCGATTTTTAGCAA 3' ist und das Bakterium als E. coli identifiziert wird.

3. Das Verfahren nach Anspruch 1, wobei das Oligonukleotid 5'CTTGCGTAAGCGCCGGGG 3' ist und das Bakterium als S. typhimurium identifiziert wird.

4. Das Verfahren nach Anspruch 1, wobei das Oligonukleotid 5'TATTCGATGCTTTAGTGC 3' ist und das Bakterium als B. subtilis identifiziert wird.

5. Verfahren zum Identifizieren von Bakterien, welches die folgenden Schritte umfaßt:
Behandeln eines makromolekularen Syntheseoperons, um einzelsträngige DNA zu bilden;
Hybridisieren eines Antisense-Oligonukleotides mit ca. 10 bis 26 Basen mit einer einzigartigen intergenischen Sequenz in der einzelsträngigen DNA des makromolekularen Syntheseoperons; und
Messen des Ausmaßes der Hybridisierung.

6. Das Verfahren nach Anspruch 5, wobei das Oligonukleotid 5'GGCCCCGATTTTTAGCAA 3' ist und das Bakterium als E. coli identifiziert wird.

7. Das Verfahren nach Anspruch 5, wobei das Oligonukleotid 5'CTTGCGTAAGCGCCGGGG 3' ist und das Bakterium als S. typhimurium identifiziert wird.

8. Das Verfahren nach Anspruch 5, wobei das Oligonukleotid 5'TATTCGATGCTTTAGTGC 3' ist und das Bakterium als B. subtilis identifiziert wird.

## Revendications

1. Un procédé d'identification de bactéries, comprenant les étapes suivantes :
hybridation d'un oligonucléotide antisens intergénique unique d'environ 10 à 26 mer sur un ARNm transcrit à partir d'un opéron de synthèse macromoléculaire ; et
mesure du degré de ladite hybridation ; dans lequel chaque oligonucléotide antisens intergénique unique ne se fixe que sur une bactérie spécifique.

2. Le procédé de la revendication 1, dans lequel l'oligonucléotide est 5' GGCCCCGATTTTTAGCAA 3' et la bactérie est identifiée comme *E. coli.*

3. Le procédé de la revendication 1, dans lequel l'oligonucléotide est 5' CTTGCGTAAGCGCCGGGG 3' et la bactérie est identifiée comme *S*. *typhimurium.*

4. Le procédé de la revendication 1, dans lequel l'oligonucléotide est 5' TATTCGATGCTTTAGTGC 3' et la bactérie est identifiée comme *B. subtilis.*

5. Un procédé d'identification de bactéries comprenant les étapes suivantes :
traitement d'un opéron de synthèse macromoléculaire pour former un ADN monobrin ;
hybridation d'un oligonucléotide antisens d'environ 10 à 26 mer avec une séquence intergénique unique contenue dans l'ADN monobrin de l'opéron de synthèse macromoléculaire ; et
mesure du degré de ladite hybridation.

6. Le procédé de la revendication 5, dans lequel l'oligonucléotide est 5' GGCCCCGATTTTTAGCAA 3' et la bactérie est identifiée comme *E. coli.*

7. Le procédé de la revendication 5, dans lequel l'oligonucléotide est 5' CTTGCGTAAGCGCCGGGG et la bactérie est identifiée comme *S. typhimurium.*

8. Le procédé de la revendication 5, dans lequel l'oligonucléotide est 5' TATTCGATGCTTTAGTGC 3' et la bactérie est identifiée comme *B. subtilis.*
